# DEMANDE DE BREVET EUROPEEN

(11) **EP 2 793 152 A1**
(43) Date de publication de la demande: **22.10.2014**
(21) Numéro de dépôt: 14165274.3
(22) Date de dépôt: 17.04.2014
(51) Int. Cl.: G06F 19/00

(54) **Méthode pour gérer un premier et un deuxième authentificateur chargés dans un dispositif de renseignement et de surveillance d'une prise d'une spécialité par un patient**

(30) Priorité: 18.04.2013 BE 201300280
(71) Demandeur: Medimind, 1325 COrroy-le-Grand (BE)
(72) Inventeur: Hiernaux, Bernard, 1325 Corroy-le-Grand (BE); Leclercqz, Frédéric, 1480 Oisquercq (BE)
(74) Mandataire: Gevers Patents

(57) **Abrégé**

Méthode pour gérer un premier et un deuxième authentificateur dans un dispositif de renseignement et de surveillance d'une prise d'une spécialité par un patient, la méthode comprenant les étapes : utiliser le premier authentificateur pour vérifier des données du patient, prélever dans une base de données les deuxièmes authentificateurs liées au premier authentificateur reprenant une liste de spécialités, adapter la spécialité et sa posologie, envoyer un ensemble des données reprenant la liste adaptée des deuxièmes authentificateurs à la centrale de gestion, préparer sur base des deuxièmes authentificateurs et par la centrale de gestion une séquence des alertes.

## Description

La présente invention concerne une méthode pour gérer un premier et un deuxième authentificateur chargés dans un dispositif de renseignement et de surveillance d'une prise d'une spécialité par un patient, comprenant la connexion à une base de données du dispositif ayant dans une première partie d'une mémoire des données du patient reprises dans le premier authentificateur.

Un nombre très élevé de personnes sont obligées de prendre un ou plusieurs médicaments par jour pour gérer un ou plusieurs problèmes de santé. Le suivi d'un traitement, gérer les prises d'un ou plusieurs médicaments et leur posologie, d'une manière optimale implique une forte discipline et constance de la part du patient. De même un suivi régulier de la part du médecin est nécessaire pour suivre les résultats de l'évolution d'une maladie et/ou de la guérison, surtout dans les cas de maladies graves. Le problème ne concerne pas uniquement des médicaments prescrits par un médecin ou le corps médical mais il concerne également d'autres substances qu'un patient peut prendre, on utilisera donc dans la suite du texte le mot spécialité pour indiquer tant un médicament soumis ou non à prescription, qu'un produit délivré en officine et non enregistré comme médicament.

La demande de brevet US 2013/0085767 divulgue une méthode et un dispositif électronique de gestion d'un traitement médical qui aident le patient sous traitement à gérer son traitement. Le patient est renseigné par un calendrier électronique de l'heure à laquelle il ou elle doit prendre un médicament. Ainsi le patient reçoit un rappel qui lui aide à respecter son traitement.

Dans la méthode suivant l'art antérieur le patient connecte le dispositif électronique à un réseau et décharge d'une base de donnés une ou plusieurs listes de spécialités prescrites ou non par le médecin, ainsi que le calendrier pour programmer la fréquence des prises des spécialités en sélectionnant l'heure et le jour de la semaine. Le patient peut choisir le traitement et la posologie de sa préférence, combiner différents traitements, sélectionner la marque d'une spécialité.

La méthode divulguée fonctionne sous un régime de maître-esclave dont le maître est le patient et l'esclave est la base de données d'où le patient décharge l'information nécessaire à la programmation du dispositif. Le fait que le patient a la possibilité de programmer et de gérer le dispositif pose un problème pour respecter la prescription du médecin, ceci principalement dans les cas où la réussite du traitement dépend fortement de son suivi conformément à sa prescription.

Il y a donc un besoin de veiller à ce que le patient ne puisse pas manipuler les informations relatives au traitement.

L'invention a pour but de proposer une méthode qui permette de programmer le dispositif indépendamment du patient tout en garantissant le lien entre le patient et sa posologie.

A cette fin la méthode suivant l'invention comprend la connexion à une base de données du dispositif ayant dans une première partie d'une mémoire des données du patient reprises dans le premier authentificateur,
ladite méthode est caractérisée en ce qu'elle comprend les étapes de :
- vérifier au niveau d'une centrale de gestion reliée à la base de données que le premier authentificateur est présent dans la base de données ;
- communiquer à partir de la centrale de gestion à une interface logée au près d'une personne, qui veille à la santé du patient, que le premier authentificateur est stocké dans le dispositif ;
- utiliser le premier authentificateur pour vérifier les données du patient, et pour prélever dans la base de données une liste des deuxièmes authentificateurs liée au premier authentificateur et reprenant une liste de spécialités dans la base de données ;

- communiquer à la personne à partir de la centrale de gestion la liste des deuxièmes authentificateurs ;
- adapter par ladite personne la ou les spécialités et leur posologie, et envoyer un ensemble des données reprenant la liste adaptée des deuxièmes authentificateurs à la centrale de gestion ;
- préparer sur base des deuxièmes authentificateurs adaptés et par l'intermédiaire de la centrale de gestion une séquence des alertes à produire par le dispositif, et stocker la séquence des alertes dans la base de données ;
- transmettre par la centrale de gestion la séquence des alertes à l'interface ;
- synchroniser par l'intermédiaire de la centrale de gestion l'ensemble de données et la séquence des alertes stockées dans la base de données avec le dispositif, et ensuite communiquer à l'interface que le dispositif est prêt à l'utilisation.
La méthode selon l'invention fonctionne sous un régime de maître-esclave dont le maître est maintenant formé par la central de gestion et l'interface logée auprès de la personne. L'esclave est maintenant le patient qui n'a pas accès à la central et donc à la programmation du dispositif.

Avantageusement, la méthode suivant l'invention est caractérisée en ce que si le premier authentificateur n'est pas repris dans la base de données, ni dans le dispositif, la personne va authentifier le patient et transmettre les données du patient par l'interface à la centrale de gestion, la central de gestion forme ensuite à l'aide des données du patient le premier authentificateur et le stocke dans la base de données. L'authentification du patient permet d'initialiser l'activation du dispositif et de le lier de façon unique au patient à qui la spécialité à prendre a été prescrite. Ainsi, la personne peut s'assurer que le patient à qui appartient le dispositif et celui à qui la spécialité a été prescrite, sont bien la même personne. De plus, la personnalisation du dispositif permet également de mieux respecter les exigences en matière de secret médical.

Avantageusement, la méthode suivant l'invention est caractérisée en ce que la liste des deuxièmes authentificateurs est formée en y introduisant une dose et une forme galénique de la spécialité à prendre par le patient, et en y introduisant la séquence des alertes formée par le ou les moments de la journée auquel/auxquels ladite dose de la spécialité doit être prise. La forme galénique de la spécialité contribue à déterminer la quantité de la spécialité à prendre pour répondre à la dose prescrite et garantir l'effet voulu par le traitement.

Avantageusement, la méthode suivant l'invention est caractérisée en ce que la liste des deuxièmes authentificateurs est formée en y introduisant également la quantité de chaque spécialité qui a été remise au patient, et en ce que l'on produit un signal de renouvellement lorsque la quantité restante de la dose de spécialités est inférieure à une quantité prédéterminée. Ceci permet au patient de faire à temps le nécessaire pour qu'il ne lui manque pas une ou plusieurs spécialités qui lui ont été prescrites.

Avantageusement, la méthode suivant l'invention est caractérisée en ce qu'une liste de validations est formée à partir de la liste des deuxièmes authentificateurs et de la validation faite par le patient chaque fois qu'il a pris la spécialité, ladite liste est stockée dans le dispositif. Comme le patient est appelé à confirmer la prise de la dose indiquée, le dispositif peut non seulement stocker l'information que la dose a effectivement été prise, mais également avec l'accord du patient le communiquer à la personne qui peut ainsi mieux suivre le respect de la posologie par le patient.

L'invention sera maintenant décrite plus en détails à l'aide des dessins qui illustrent une forme de réalisation préférée d'un réseau de communication et du dispositif de renseignement et de surveillance du suivi de la prise d'une spécialité (sur base des posologies) prescrite ou recommandée à un patient. Dans les dessins :
la figure 1 illustre de façon schématique le réseau de communication et ses différents composants;
la figure 2 illustre de façon schématique comment la communication est réalisée dans le réseau de communication;
la figure 3 illustre l'activation du dispositif de renseignement et de surveillance du suivi de la prise d'une spécialité (sur base de posologies) prescrite ou recommandée à un patient;
la figure 4 illustre le chargement de la posologie dans le dispositif; et
la figure 5 illustre le fonctionnement du dispositif.

Dans les dessins une même référence a été attribuée à un même élément ou à un élément analogue.

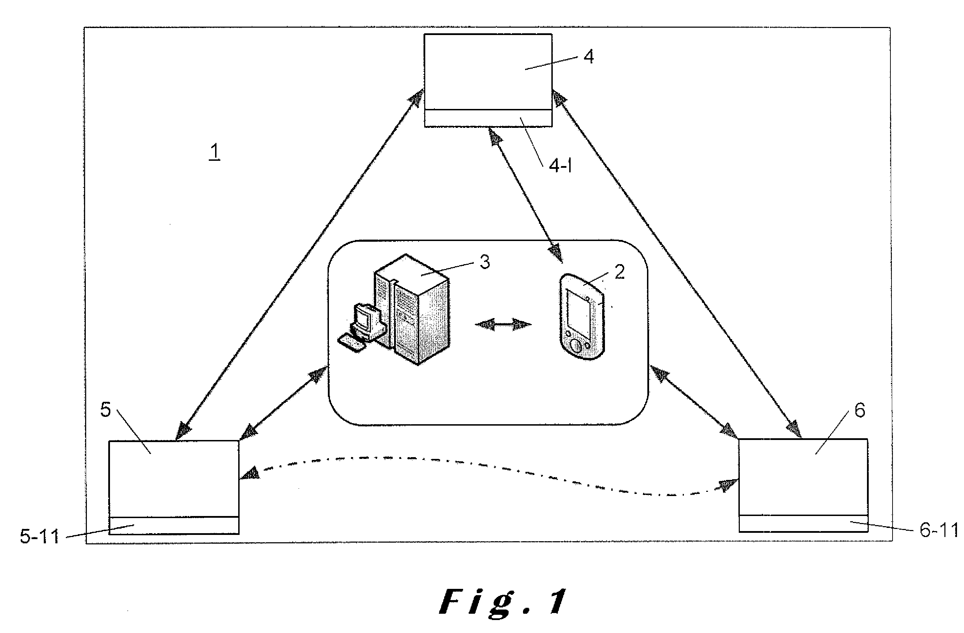

La figure 1 illustre un exemple d'un réseau de communication 1 d'information concernant des spécialités et leurs posologies d'un premier ensemble de patients. Dans ce réseau trois personnes jouent un rôle important, à savoir le patient 4, son médecin traitant 5 et son pharmacien 6. Bien entendu, le médecin 5 n'est pas nécessairement formé par une seule personne et comporte l'ensemble de médecins ou autres personnes, qui veillent à la santé du patient 4, en particulier le généraliste, le ou les spécialistes et le dentiste. Il en va de même pour le pharmacien 6 qui peut non seulement être le pharmacien usuel du patient, mais par exemple également le pharmacien de garde.

Dans le réseau de communication 1 suivant l'invention chaque patient 4 possède un dispositif 2 de renseignement et de surveillance de la prise d'une spécialité par un patient 4. Pour des raisons de secret médical, le dispositif 2 est chaque fois lié à un patient unique. Le dispositif 2 est en liaison avec une centrale de gestion 3, qui est logée auprès du gestionnaire du réseau de communication. Le médecin 5 possède une première interface 5-11 et le pharmacien 6 possède une deuxième interface 6-11. La première et la deuxième interface permettent d'établir une communication entre le dispositif 2, le médecin 5, respectivement le pharmacien 6 et la centrale de gestion 3. Ces interfaces 11 peuvent par exemple être formées par un ordinateur relié à l'internet ou par un téléphone. Le dispositif 2 est quant à lui muni d'un organe de communication, qui est soit entièrement intégré dans le dispositif 2, soit coopère avec un ordinateur relié à l'internet ou à un téléphone. L'organe de communication permet la communication avec la première et la deuxième interface ainsi qu'avec la centrale de gestion 3.

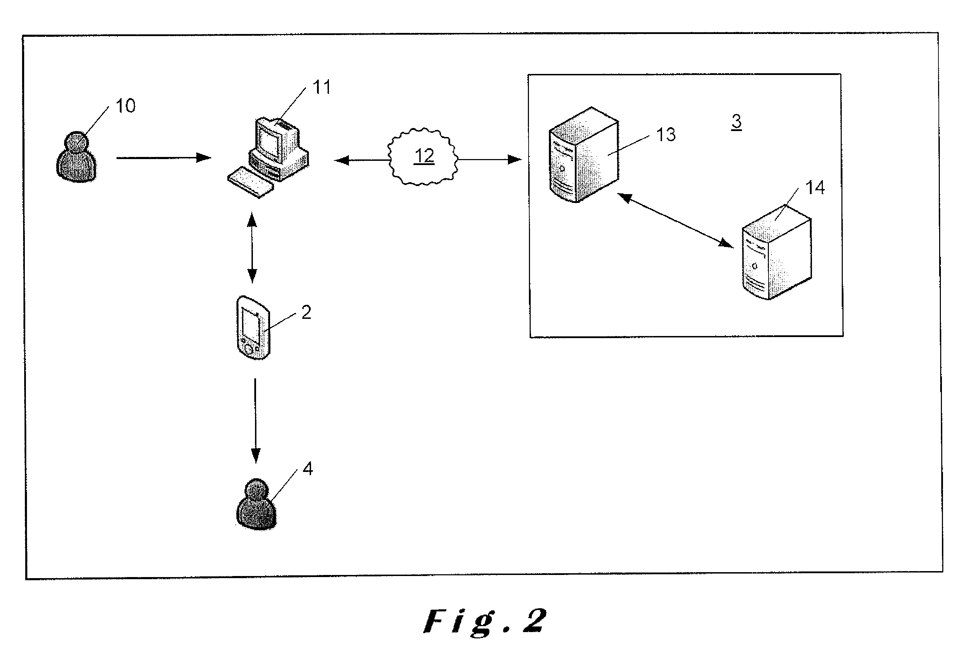

La figure 2 illustre de façon schématique comment la communication est réalisée dans le réseau de communication 1 suivant l'invention. Un utilisateur 10, qui est soit le médecin 5, soit le pharmacien 6 du patient 4 auquel appartient le dispositif 2, peut entrer en communication avec le dispositif 2 à l'aide de son interface 11. Cette dernière est reliée à la centrale de gestion 3, par exemple via l'internet ou le téléphone 12. La centrale de gestion 3 comporte de préférence un serveur d'application 13 et une base de données 14. Cette dernière est agencée pour stocker d'une part des données permettant d'authentifier les patients titulaires d'un dispositif 2 et d'autre part des données relatives aux spécialités et leurs posologies prises par chacun de ces patients. La communication entre le dispositif 2 et l'interface 11 peut se faire par exemple à l'aide d'un câble ou sans fil. La communication par câble permet de mieux sauvegarder la confidentialité des données transmises, mais il sera clair que lorsque la communication est réalisée sans fil, la confidentialité peut être sauvegardée en encryptant les données.

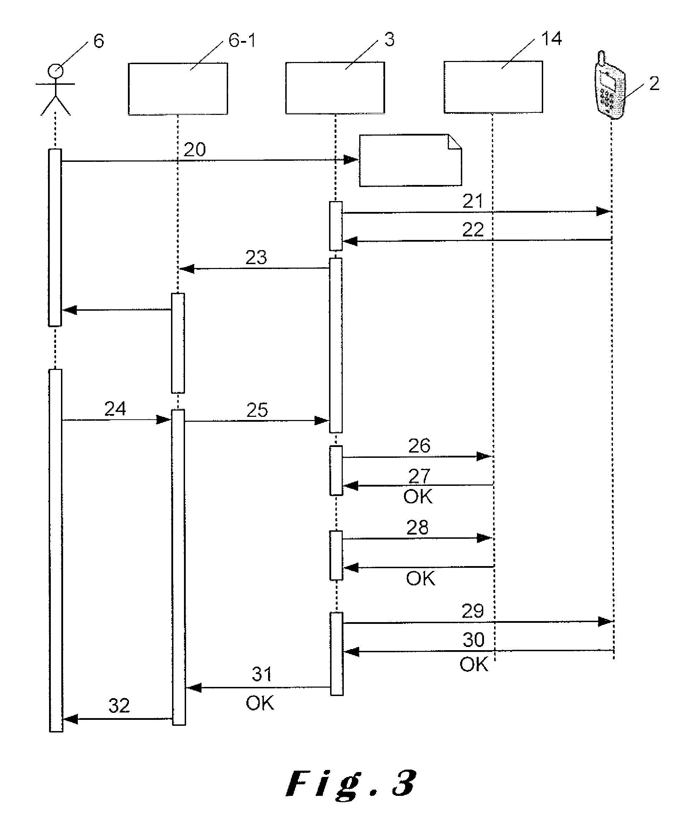

La figure 3 illustre l'activation du dispositif 2 de renseignement et de surveillance. Lorsqu'un patient 4 à qui son médecin 5 lui a prescrit une ou plusieurs spécialités et leur posologie veut disposer d'un dispositif 2 de renseignement et de surveillance du suivi de la prise d'une spécialité (sur base des posologies), il peut par exemple se rendre chez son pharmacien 6 pour faire activer son dispositif 2. Bien entendu le médecin 5 pourrait également se charger de cette activation. Cette activation est importante car elle permet de personnaliser le dispositif 2 et de le lier au patient 4 à qui la spécialité à prendre a été prescrite. Ainsi, le médecin 5 et le pharmacien 6 peuvent s'assurer que le patient 4 à qui appartient le dispositif 2 et celui à qui la spécialité a été prescrite sont bien la même personne. De plus, la personnalisation du dispositif 2 permet également de mieux respecter les exigences en matière de secret médical.

Le dispositif 2 comporte une mémoire ayant une première et une deuxième partie. La première partie est agencée pour y stocker un premier authentificateur authentifiant le patient pour lequel la ou les spécialités est destinée. La deuxième partie est agencée pour y stocker une liste de deuxième authentificateurs, lesquels deuxièmes authentificateurs authentifient chacun une dose et une forme galénique d'une spécialité à prendre par le patient 4 ainsi que le ou les moments de la journée auquel/auxquels ladite dose de la spécialité doit être prise par le patient 4. Par exemple pour le patient A il sera prescrit que chaque matin de préférence à une heure précise il doit prendre une tablette de 5mg d'une spécialité B.

Lorsque le patient 4 s'est acheté un dispositif 2 suivant l'invention, son pharmacien 6 ou son médecin 5 va à l'aide de son interface 11 se mettre en contact avec la centrale de gestion 3. Pour des raisons de clarté, le reste de la description sera décrit en partant de l'exemple que c'est le pharmacien 6 qui procède à l'initialisation. Le pharmacien 6 va connecter 20 le dispositif 2 à son interface 11 et lancer la communication avec la centrale de gestion 3. La centrale de gestion 3 va ensuite demander 21 au dispositif 2 de s'authentifier. Comme le dispositif 2 n'est pas encore initialisé il ne peut s'authentifier et envoie 22 un message à l'interface 11 indiquant cela. La centrale 3 va alors lancer le processus d'initialisation pour un nouveau patient 4 et demander 23 au pharmacien 6 de faire connaître le patient 4, par exemple à l'aide de son nom et prénom, date de naissance, adresse, etc. Toutes ces données forment alors la base pour former un premier authentificateur. Après que le pharmacien 6 a communiqué 24 toutes les données du patient, elles sont envoyées 25 à la centrale de gestion 3 qui va les stocker 26 dans la base de données 14. Lorsque les données sont reçues et stockées, la centrale de gestion 3 envoie 27 alors une confirmation. Ensuite la centrale 3 va produire 28 le premier authentificateur et le stocker dans sa base de données 14. Ensuite le premier authentificateur sera transmis 29 au dispositif 2 qui va le stocker dans la première partie de la mémoire. Après cela le dispositif 2 va communiquer 30 à la centrale 3 que le premier authentificateur a été stocké dans sa mémoire. Lorsque le stockage tant dans la base de données 14 que dans la première partie de la mémoire a été effectué avec succès, la centrale de gestion 3 produit 31 une information comme quoi l'enregistrement a réussi et elle en informe 32 le pharmacien 6.

Le cas échéant le patient pourrait également initialiser son dispositif 2 par l'usage de l'internet en se connectant au site de la centrale 3.

Lors de cette visite ou d'une prochaine visite auprès de son médecin 5 ou de son pharmacien 6, le patient 4 lui remettra alors son dispositif 2. A l'aide de son interface 11 et du dispositif 2 du patient, le médecin 5 ou le pharmacien 6 pourra alors entrer en communication avec la centrale de gestion 3. Le médecin 5 ou le pharmacien 6 pourra aussi vérifier, à l'aide du premier authentificateur que le dispositif 2 appartient effectivement au patient 4.

Après que le médecin 5 a effectué la consultation du patient et qu'il estime que le patient 4 a besoin d'un traitement, il va prescrire une ou plusieurs spécialités et leur posologie. Cette ou ces spécialités et leur posologie doivent alors être communiquées à la centrale de gestion 3 par le médecin 5 pour y être stockée dans la base de données 14. La posologie et la ou les spécialités prescrites seront associés au premier authentificateur et seront stockés dans la base de données 14. Bien entendu le médecin 5 peut également inscrire la ou les spécialités et leur posologie sur une feuille de papier et la remettre au patient 4, qui la donnera ensuite à son pharmacien 6 pour que ce dernier puisse exécuter l'opération de mise en mémoire dans la base de données 14.

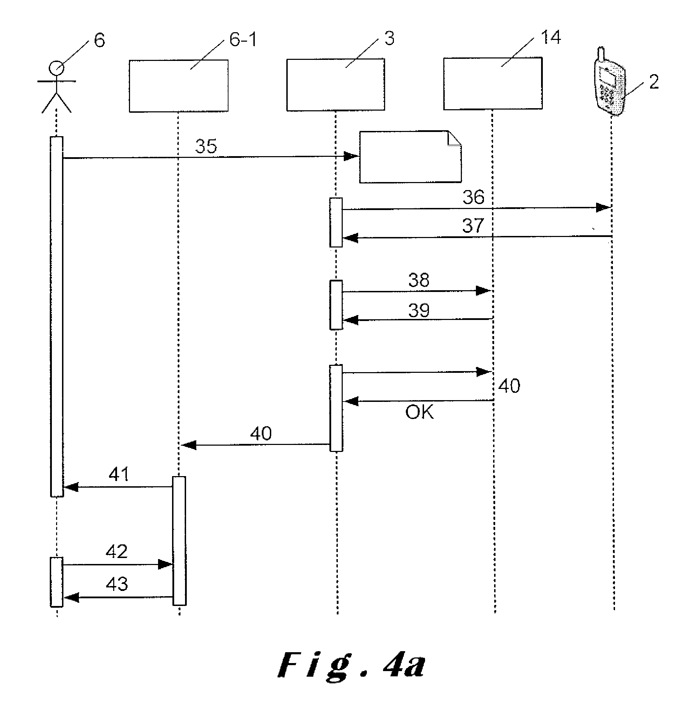

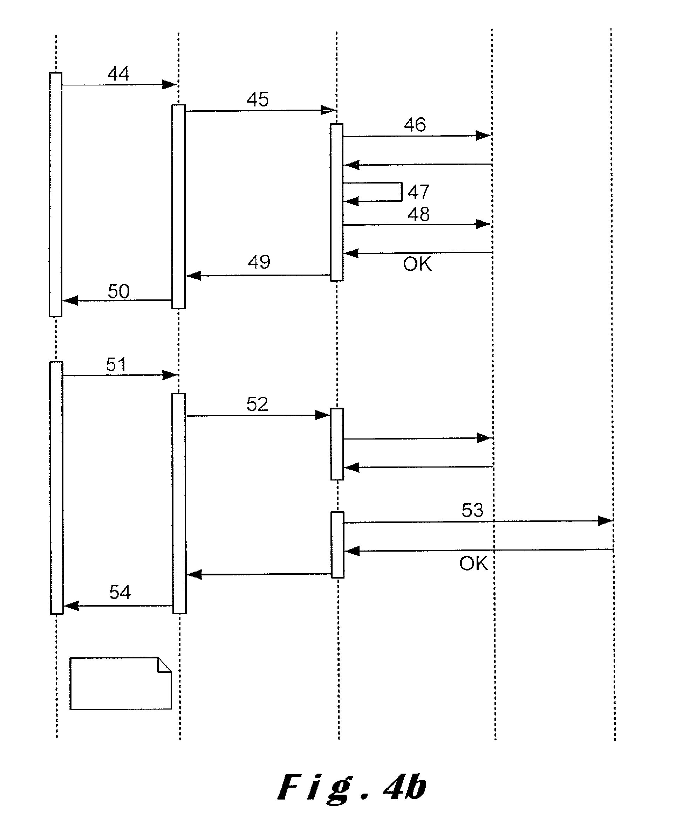

Après avoir rendu visite à son médecin 5, le patient 4 pourra se rendre auprès de son pharmacien 6 pour chercher les spécialités prescrites. Ce dernier va alors se charger de stocker la ou les spécialités et leur posologie dans la deuxième partie de la mémoire. Ici on supposera, à nouveau pour des raisons de clarté, que c'est le pharmacien 6 qui va s'occuper de stocker la ou les spécialités et leur posologie. La figure 4 illustre les différentes étapes pour charger la ou les spécialités et leur posologie dans le dispositif 2. Le patient 4 va remettre son dispositif 2 ainsi que la prescription du médecin 5 au pharmacien 6. Le pharmacien 6 va brancher 35 le dispositif 2 sur son interface 11 et établir une connexion avec la centrale de gestion 3. L'interface 11 va lire 36 le premier authentificateur dans la première partie de la mémoire du dispositif 2 et le cas échéant la liste des validations faite par le patient 4 chaque fois qu'il ou elle a pris la spécialité. Ces données seront alors envoyées 37 à la centrale 3. Cette dernière va chercher 38 dans sa base de données 14 ce premier authentificateur pour prélever les données du patient et la liste des deuxièmes authentificateurs pour les envoyer 39 à la centrale 3. Cette liste de spécialités, les validations prélevées et le premier authentificateur seront envoyés à la centrale 3. Cette dernière va alors stocker 40 la liste des validations dans la base de données 14 et vérifier les données du patient 4. Après vérification 40 les données du patient les données seront affichées 41 auprès du pharmacien 6. Après cela, le pharmacien 6 peut alors vérifier que le dispositif 2 appartient effectivement au patient 4 pour lequel la spécialité et sa posologie a été prescrite. Si tel ne serait pas le cas, la transaction sera interrompue. Ainsi le pharmacien 6 peut vérifier qu'il y a concordance entre le dispositif 2 et le patient 4 pour qui la spécialité et sa posologie a été prescrite.

Après que l'authentification a été valablement effectuée, le pharmacien 6 va demander 42 un affichage d'un formulaire pour introduire la ou les spécialités et leur posologie prescrite par le médecin 5, lequel formulaire sera alors affiché 43. Le pharmacien 6 peut alors encoder 44 dans le formulaire la ou les spécialités et leur posologie prescrite en y introduisant non seulement la marque de la spécialité, mais également sa forme galénique et la dose à prendre aux moments indiqués. Le pharmacien 6 va également encoder la quantité de chaque spécialité qu'il a remise au patient. Le cas échéant le pharmacien 6 pourrait encoder à la place de la spécialité d'une certaine marque prescrite par le médecin 5 qu'il a donné une spécialité d'une autre marque ayant bien entendu la même substance active. L'ensemble de ces données introduites par le pharmacien 6 sera alors envoyé 45 à la centrale de gestion 3 pour y être stocké 46 dans la base de données 14.

Après réception des données encodées par le pharmacien 6 et leur stockage dans la base de données 14, la centrale 3 va les traiter pour préparer 47 la séquence des alertes à produire par le dispositif 2. Cette séquence d'alertes sera stockée 48 dans la base de données 14 et transmise 49 à l'interface 11 du pharmacien 6 pour y être affichée 50. Le pharmacien 6 va alors demander 51 à centrale de synchroniser 52 la liste stockée dans la base de données 14 avec le dispositif 2 et d'inscrire 53 la liste des spécialités et la séquence des alertes dans la deuxième partie de la mémoire.

Après cette écriture dans la deuxième partie de la mémoire la centrale 3 va envoyer 54 un message au pharmacien 6 que l'écriture est terminée et qu'il peut remettre le dispositif 2 au patient 4. Bien entendu ce processus sera repris chaque fois que soit la posologie aura changé soit que la ou les spécialités ont changées ou chaque fois que le patient 4 viendra pour renouveler les spécialités auprès de son pharmacien 6.

Dans l'exemple décrit ci-dessus c'est la centrale 3 qui prépare la séquence des alertes. Bien entendu il serait également possible de prévoir le dispositif 2 avec un processeur plus puissant et la capacité de déterminer la séquence des alertes.

Le dispositif 2 suivant l'invention comporte une horloge qui est reliée à l'organe de communication du dispositif 2. Sur base de la liste des spécialités et de la séquence des alertes chargées dans la deuxième partie de la mémoire, le dispositif 2 va à l'aide de son horloge surveiller le temps afin de vérifier quand le moment est venu pour prendre une ou plusieurs spécialités. Le moment peut être une heure précise si la posologie le prescrit ainsi, mais il peut également être une plage horaire que le patient 4 ou le pharmacien 6 peut lui-même préciser, comme par exemple le matin ou le soir. Ainsi, si la spécialité doit par exemple être prise uniquement le matin, ou lors de chaque repas, le patient peut communiquer au pharmacien 6 ses plages horaires de convenance.

L'organe de communication du dispositif 2 comporte ainsi un générateur de signal relié à l'horloge. Lorsque l'horloge indique un moment qui correspond à un de ceux repris dans la séquence des alertes, le générateur de signal va le détecter et produire un signal de renseignement. Si la séquence des alertes comporte plusieurs de ces moments par jour, bien entendu le générateur de signal va détecter chacun de ces moments de la journée et produire à chaque fois un signal de renseignement. Ce dernier peut être réalisé sous plusieurs formes en fonction de l'implémentation du dispositif. Ainsi, le dispositif 2 peut être équipé d'un écran d'affichage sur lequel sera repris le ou les spécialités à prendre et la dose à prendre et le cas échéant aussi leur forme galénique. Cette affichage peut se faire par exemple en mentionnant le nom de la ou des spécialités, en reprenant une photo de la boîte et/ou de la spécialité même. Le cas échéant l'affichage peut être accompagné d'un signal sonore et/ou vibrant et/ou lumineux.

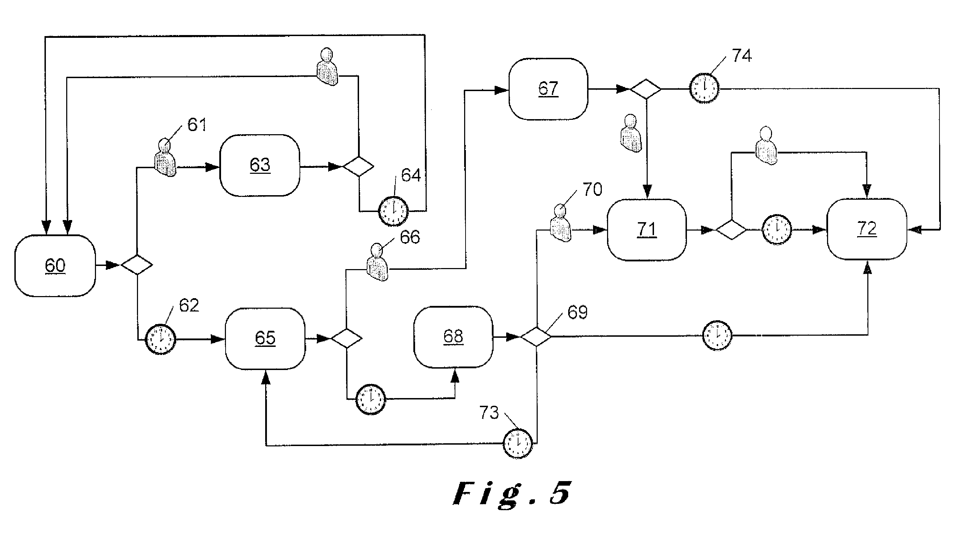

La production de ce signal de renseignement sera maintenant décrite plus en détails à l'aide de l'exemple repris à la figure 5. Le dispositif 2 est normalement en veille 60 et peut être activé soit par le patient même 61 ou par une personne d'un service médical, soit par l'horloge 62 qui coopère avec la liste des spécialités et la séquence des alertes stockée dans la mémoire. Lorsque le patient ou ladite personne a activé le dispositif ce dernier va afficher 63 les spécialités et les plages horaires stockées et retourner 64 en veilleuse après un temps prédéterminé. Si par contre c'est l'horloge qui a activé le dispositif 2 un signal de renseignement sonore et visuel sera produit 65. Si le patient appuie 66 sur une touche d'arrêt prévue à cette fin, le signal sonore va s'arrêter 67 pour ne pas incomber le patient et seul l'affichage sera maintenu. Si le patient n'arrête pas lui-même le signal sonore, ce dernier s'éteint 68 après un temps prédéterminé de par exemple 40 secondes. Le dispositif va ensuite vérifier 69 si le patient valide 70 qu'il a bien pris la ou les spécialités reprises dans le message affiché. Si tel est le cas le dispositif va mémoriser 71 que le patient lui a indiqué qu'il a pris ces spécialités et le dispositif se met en veilleuse 72. Si par contre le patient n'a pas informé le dispositif qu'il a pris ses spécialités le dispositif va à nouveau produire un signal sonore 73 pour rappeler au patient qu'il doit valider la prise des spécialités. Ce dernier rappel est répété à plusieurs reprises afin que le patient puisse valider la prise des spécialités. Si le patient ne valide pas la prise, la production du signal sera arrêtée 74 et il sera enregistré dans la deuxième partie de la mémoire que le patient n'a pas validé la prise.

En désactivant le générateur d'appel, le patient indique au dispositif 2 qu'il a reçu le message comme quoi il devait prendre sa ou ses spécialités et qu'il ou elle les a effectivement pris. La désactivation du générateur d'appel est stockée dans la mémoire, par exemple dans la deuxième partie ou dans une autre partie réservée à cette fin. Ainsi, le dispositif 2 peut mémoriser la réponse fournie par le patient.

Le fait que la réponse fournie par le patient est stockée dans la mémoire du dispositif va permettre ultérieurement au médecin 5 de mieux vérifier que le patient 4 a effectivement respecté la posologie que le médecin 5 lui avait prescrite. Même si le patient 4 valide la prise de la spécialité sans l'avoir effectivement prise, le fait de devoir valider le générateur d'appel va responsabiliser le patient 4 qui voudra face à son médecin 5 garder une bonne conscience et ne sera donc pas tenter à fausser l'information. De plus, le patient 4 sait que le médecin 5 peut facilement à l'aide d'une prise de sang ou d'urine vérifier s'il a effectivement pris la dose prescrite. Le dispositif 2 suivant l'invention permet ainsi au médecin 5 de mieux surveiller son patient 4 et ainsi d'être mieux informé que les spécialités qu'il a prescrites portent effectivement leur effet.

Le fait que la réponse fournie par le patient 4 est stockée dans la mémoire du dispositif 2 permet également de comparer, à l'aide d'un organe de comparaison relié à la mémoire et au générateur d'information, le nombre de spécialités pris avec le nombre remis par le pharmacien 6. Ainsi, l'organe de comparaison peut produire un signal de renouvellement lorsque la quantité restante est inférieure à une quantité prédéterminée, ce qui permet au patient 4 de renouveler à temps sa dose de spécialités.

Le dispositif 2 comporte également une commande d'intervention qui permet, par exemple lors d'un accident ou lors d'une intervention médicale d'urgence, au personnel qui va soigner le patient, de connaître les spécialités que le patient prend. En effet comme la liste des spécialités est stockée dans la mémoire, une activation de cette commande d'intervention va permettre une lecture de la mémoire et un affichage de la liste des spécialités, posologies et dosages de même que certaines informations spécifiquement voulues par le patient et encodées par le pharmacien comme par exemple le numéro de téléphone d'un proche ou de son médecin, facteur rhésus. Ceci permet alors audit personnel de connaître exactement les spécialités du patient et de pouvoir agir en connaissance de cause.

## Revendications

1. Méthode pour gérer un premier et un deuxième authentificateur chargés dans un dispositif (2) de renseignement et de surveillance d'une prise d'une spécialité par un patient (4), laquelle méthode comprend la connexion (35) à une base de données (14) du dispositif (2) ayant dans une première partie d'une mémoire des données du patient reprises dans le premier authentificateur ;
ladite méthode est **caractérisée en ce qu'**elle comprend les étapes de :
- vérifier au niveau d'une centrale de gestion (3) reliée à la base de données (14) que le premier authentificateur est présent dans la base de données (14) ;
- communiquer (40) à partir de la centrale de gestion (3) à une interface (11) logée au près d'une personne, qui veille à la santé du patient, que le premier authentificateur est stocké dans le dispositif (2) ;
- utiliser (38) le premier authentificateur pour vérifier les données du patient (4), et pour prélever dans la base de données (14) une liste des deuxièmes authentificateurs liée au premier authentificateur et reprenant une liste de spécialités dans la base de données (14) ;
- communiquer (41) à la personne à partir de la centrale de gestion (3) la liste des deuxièmes authentificateurs ;
- adapter (44) par ladite personne la ou les spécialités et leur posologie, et envoyer (45) un ensemble des données reprenant la liste adaptée des deuxièmes authentificateurs à la centrale de gestion (3) ;
- préparer (47) sur base des deuxièmes authentificateurs adaptés et par l'intermédiaire de la centrale de gestion (3) une séquence des alertes à produire par le dispositif (2), et stocker (48) la séquence des alertes dans la base de données (14) ;
- transmettre (49) par la centrale de gestion la séquence des alertes à l'interface (11) ;
- synchroniser (52) par l'intermédiaire de la centrale de gestion (3) l'ensemble de données et la séquence des alertes stockées dans la base de données (14) avec le dispositif (2), et ensuite communiquer (54) à l'interface (11) que le dispositif (2) est prêt à l'utilisation.

2. Méthode selon la revendication 1, **caractérisée en ce que** si le premier authentificateur n'est pas repris dans la base de données (14), ni dans le dispositif (2), la personne va authentifier le patient (4) et transmettre (20) les données du patient par l'interface (11) à la centrale de gestion (3), la central de gestion (3) forme (23) ensuite à l'aide des données du patient le premier authentificateur et le stocke (26) dans la base de données (14).

3. Méthode selon les revendications 1 ou 2, **caractérisée en ce que** la liste des deuxièmes authentificateurs est formée en y introduisant une dose et une forme galénique de la spécialité à prendre par le patient (4), et en y introduisant la séquence des alertes formée par le ou les moments de la journée auquel/auxquels ladite dose de la spécialité doit être prise.

4. Méthode selon la revendication 3, **caractérisée en ce que** la liste des deuxièmes authentificateurs est formée en y introduisant également la quantité de chaque spécialité qui a été remise au patient (4), et **en ce que** l'on produit un signal de renouvellement lorsque la quantité restante de la dose de spécialités est inférieure à une quantité prédéterminée.

5. Méthode selon les revendications 1 à 4, **caractérisée en ce que** l'on détecte et vérifie par l'intermédiaire d'une horloge à partir de la séquence des alertes le ou les moments de prendre une ou plusieurs spécialités reprises dans la liste de spécialités.

6. Méthode selon la revendication 5, **caractérisée en ce que** l'on produit un signal de renseignement à partir d'un générateur de signal relié à l'horloge quand le ou les moments de prendre une spécialité sont détectés.

7. Méthode selon l'une des revendications précédentes, **caractérisée en ce qu'**une liste de validations est formée à partir de la liste des deuxièmes authentificateurs et de la validation faite par le patient (4) chaque fois qu'il a pris la spécialité, ladite liste est stockée dans le dispositif (2).

8. Méthode selon les revendications 6 ou 7, **caractérisée en ce que** si le patient (4) ne valide pas la prise de la spécialité le générateur va à nouveau produire un signal pour rappeler au patient (4) qu'il doit valider la prise des spécialités de renseignement.

9. Méthode selon la revendication 8, **caractérisée en ce que** si le patient (4) ne valide pas la prise après une nouvelle production du signal, la production du signal sera arrêtée et il sera enregistré dans la deuxième partie de la mémoire que le patient (4) n'a pas validé la prise.

10. Méthode selon l'une des revendications précédentes, **caractérisée en ce que** l'on affiche le nom de la ou les spécialités à prendre, en reprenant une photo de la boîte et/ou de la spécialité même ou des spécialités mêmes.

11. Méthode selon l'une des revendications précédentes, **caractérisée en ce que** l'on communique à la personne en communicant soit à un médecin traitant, soit un ensemble de médecins, soit un généraliste, soit le ou les spécialistes, soit le dentiste, soit le pharmacien usuel du patient, soit le pharmacien de garde.

12. Méthode selon l'une des revendications précédentes, **caractérisée en ce que** l'on prend en tant que la spécialité soit un médicament soumis ou non à prescription, soit un produit délivré en officine en non enregistré comme médicament.
